# EUROPÄISCHE PATENTSCHRIFT

(11) **EP 2 497 765 B1**
(45) Veröffentlichungstag und Bekanntmachung des Hinweises auf die Patenterteilung: **11.11.2015**
(21) Anmeldenummer: 10828601.4
(22) Anmeldetag: 28.09.2010
(51) Int. Cl.: C07C 277/08, C07C 279/10, C07C 279/12, C07K 5/062

(54) **VERFAHREN ZUR HERSTELLUNG VON KREATINAMIDEN**
PROCESS FOR PREPARING CREATINE AMIDES
PROCÉDÉ DE PRODUCTION D'AMIDES DE CRÉATINE

(30) Priorität: 03.11.2009 RU 2009140380
(43) Veröffentlichungstag der Anmeldung: 12.09.2012
(73) Patentinhaber: Zakrytoe Aktsionernoe Obschshestvo "Vertex", St.Petersburg 199106 (RU)
(72) Erfinder: BUROV, Sergej Vladimirovich, St.Petersburg 197198 (RU); VESELKINA, Olga Sergeevna, St.Petersburg 199004 (RU); LEKO, Maria Victorovna, St.Petersburg 195427 (RU)
(74) Vertreter: Jeck, Anton
(86) Internationale Anmeldenummer: PCT/RU2010/000534
(87) Internationale Veröffentlichungsnummer: WO 2011/056091

(56) Entgegenhaltungen:
- DE-A1- 2 304 591
- RU-C1- 2 354 645
- US-A- 3 972 872
- S. BUROV, ET AL.: "Creatinyl amino acids-new hybrid compounds with neuroprotective activity", JOURNAL OF PEPTIDE SCIENCE., Bd. 17, Nr. 9, 1. September 2011 (2011-09-01), Seiten 620-626, XP055021476, John Wiley and Sons Ltd., Chichester, GB ISSN: 1075-2617, DOI: 10.1002/psc.1379

## Beschreibung

Die Erfindung bezieht sich auf das Gebiet der pharmakologischen Chemie, nämlich auf ein Verfahren zur Herstellung von biologisch aktiven Substanzen (BAS), insbesondere Kreatinamiden.

Kreatin (Kr) ist ein endogener Nährstoff. Er ist in unterschiedlichen Geweben von Säugetieren, z. B. in der Leber, in den Nieren, im Muskelgewebe, im Hirngewebe, im Blut sowohl in freier Form als auch als Kreatinphosphat immer vorhanden. Kreatin wird als Mittel zur Verbesserung des energetischen Gewebemetabolismus betrachtet. Es erhöht die energetische Reserve von ATP (Adenosintriphosphat), vor allem in Muskel- und Nervenzellen.

Kreatinphosphat unterstützt den Adenosintriphosphat-Spiegel bei der Erhöhung des Energieverbrauchs in der Zelle. Das heißt, es nimmt am physiologischen Vorgang der Phosphorylierung von Adenosindiphosphat unter Bildung von Adenosintriphosphat teil. Neben Glykogen ist Kreatinphosphat eines der Hauptquellen des Umwandlungszyklus von hochenergetischen Phosphaten und ist somit in der oxidativen Glukose-Phosphorylierung beteiligt. Das stellt die Energieabgabe sicher, die für die Funktion der Muskelgewebezellen, einschließlich der Skelettmuskeln und des Herzmuskels, erforderlich ist. Da das Kreatinphosphat die Biosynthese von Adenosintriphosphat sicherstellen kann, steigert die Vergrößerung des Kreatingehalts in den Muskeln die Muskelvorräte an Kreatinphosphat, verbessert den Energie-Metabolismus im Gewebe, verbessert die Arbeitsfähigkeit (Ausdauerfähigkeit) der Muskeln und vergrößert die Muskelmasse.

Kreatinphosphat und Kreatin sind auch allosterische Regler bei Zellenvorgängen (N. Brustovetsky et al., J. of Neurochemistry, 2001, 76, 425-434). So führt die tägliche Einnahme von 20 - 30 g Kreatin-Monohydrat im Laufe von einigen Tagen zu einer mehr als 20%igen Steigerung des Gesamtkreatingehalts in den menschlichen Skelettmuskeln. Diese Wirkung ist besonders attraktiv im Zusammenhang mit der Möglichkeit, Kreatin als Nahrungsergänzungsmittel zur Stärkung des Organismus und zur Erhöhung der Arbeitsfähigkeit zu gebrauchen. So wird die Anwendung von Kreatin-Monohydrat in einer Tagesdosis von 15 g innerhalb von mindestens zwei Tagen zur Vergrößerung der Muskelkraft verwendet (WO 94/02127, 1994).

Kreatin und Kreatinphosphat werden weit verbreitet in der Medizin eingesetzt. So werden Kreatin, Kreatinphosphat und Zyklokreatin (US 6 706 764, 2004) bei der Behandlung von Nervenerkrankungen empfohlen. Dazu zählen z. B. diabetische Krankheit und Neuropathie, Alzheimer-Krankheit, Parkinson-Krankheit, Insult usw. sowie solche Stoffwechselstörungen wie Hyperglykämie und Zuckerkrankheit (US 6 193 973, 2001). Die perorale Anwendung von Kreatin ist für die Behandlung von Herzinsuffizienz (WO/EP 97/06225, 1999) und Asthma (US 6 093 746, 2000) beschrieben. Die Verwendung von Kreatinphosphat ist bei der Behandlung von Herz-Kreislaufsystemerkrankungen indiziert. Seine Anwendung ist auch bei der Behandlung von Neubildungen als aussichtsreich (US 5 219 846, 1993) nachgewiesen.

Jedoch ist die Anwendung von Kreatin und Kreatinphosphat durch die geringe Löslichkeit und die Instabilität in Wassermedien bei physiologisch gerechten pH-Werten (RU 2 295 261, 2007) eingeschränkt.

Darüber hinaus lässt sich Kreatin aus dem Magen-Darm-Kanal nur schlecht absorbieren. Der Absorptionsgrad beträgt 1 - 14%. Dadurch ist die Anwendung von hohen Kreatindosen nötig. Um den Gebrauch von Kreatin wirksam zu machen, müssen die zurzeit hergestellten Zusammensetzungen zu je 20 g pro Tag eingenommen werden. Darüber hinaus kann die Einführung von hohen Kreatindosen neben der Steigerung der Therapiekosten auch negative Auswirkungen für den Körper haben. Das können z. B. Störung des Stickstoffwechsels, Magen-Darm-Störungen, Durchfall usw. sein.

In diesem Zusammenhang wird die Herstellung von Kreatin-Derivaten von besonders großem Interesse. Sie weisen eine längere Haltbarkeit und eine höhere Bioaktivität auf. Damit wird es möglich, einerseits die Dosis der zu verwendenden Substanz zu verringern und andererseits neue Anwendungsgebiete zu entdecken.

Das größte Interesse erwecken dabei die Derivate von Kreatin und verschiedenen organischen Säuren. Aus dem Stand der Technik ist z. B. der Gebrauch von Pyruvaten des Kreatins bekannt (US 6 166 249, 2000; RU 2 114 823, 1998). Sie dienen zur Erhöhung der Arbeitsfähigkeit, zur Körpergewichtsabnahme und zur Anpassung an die Sauerstoffinsuffizienz bei Ischämie. Sie werden auch als Nahrungsergänzungsmittel, als Hautschutzmittel gegen Alterung und gegen die Einwirkung von Sonnenstrahlen (US 7 186 754, 2007) sowie als Heilmittel bei der Behandlung von Frauengeschlechtskrankheiten, insbesondere von Dysmenorrhoe (US 6 503 951, 2000), verwendet.

Derivate von und der Malon-, Malein-, Fumar- und Orotsäuren sowie Taurin (CN 10/249338, 2003; US 6 861 554, 2005; US 6 166 249, 2000; CA 10/740263, 2003) sind als Diättherapie in Form von Nahrungszusatzstoffen indiziert. Kreatinzitrat (US 2004/077719, 2004) wird als nootropisches Mittel empfohlen.

Die Erfinder haben einige der aussichtsreichen Kreatin-Derivate synthetisiert und untersucht. Diese sind Kreatinamide mit der allgemeinen Formel: NH=C(NH₂)-N(CH₃)-CH₂-CO-NH-R*X, wobei R ein Aminosäurerest oder ein substituierter Aminosäurerest und X eine organische Säure oder Mineralsäure oder Wasser ist (RU 2 354 645, 2009).

Während der Versuche ist Folgendes festgestellt worden: die synthetisierten Kreatinamide weisen eine höhere Löslichkeit und Haltbarkeit in Wasserlösungen gegenüber dem bekannten Stand der Technik auf. Daher können sie viel breiter als Kreatinquelle im Organismus benutzt werden.

Das dem erreichten Endeffekt nach am nächsten liegende Verfahren zur Erzeugung von ähnlichen Kreatinamiden beruht auf der Zusammenwirkung von freien bzw. geschützten guanidinierenden Wirkstoffen mit Sarkosinamiden in polaren organischen Lösungsmitteln bei einer Temperatur von max. 50° C. Auf der Grundlage von Sarkosinamiden können auch andere Derivate der Aminosäuren unter Einsatz von typischen in der Fachliteratur beschriebenen chemischen Reaktionen hergestellt werden (A.A. Gershkovich, V.K. Kibirev "Synthese der Peptide. Reagens und Verfahren". Kiev, "Naukova Dumka", 1987).

Der Mangel dieses Verfahrens ist seine Mehrstufigkeit. Sie hängt damit zusammen, dass die Sarkosinamide im Voraus hergestellt werden müssen und dass die Ausbeute des Zielprodukts nicht hoch genug ist. So beträgt das Zielprodukt beim Kreatinil-Glyzin von Benzylester-Hydrochlorid etwa 5%.

Es wird ein einfacheres und wirksameres Verfahren zur Herstellung von Kreatinamiden mit höherer Ausbeute vorgeschlagen. Das wird durch die Verwandlung von Kreatin (Kr) in einen in organischen Lösungsmitteln löslichen Komplex erreicht. Dafür wird Kreatin mit p-Toluol-Sulfonsäure bis zum vollständigen Aufschluss von Kreatin behandelt. Danach erfolgt eine Zusammenwirkung dieses Komplexes mit Derivaten von Aminosäuren, die eine primäre bzw. sekundäre Aminogruppe enthalten. Es geht dabei insbesondere um Ester- oder Amid-Derivate von aliphatischen oder aromatischen Aminosäuren unter konsequent eingebrachten kondensierenden Wirkstoffen und einer Base.

Die p-Toluol-Sulfonsäure (p-TSS) wird mindestens in einer äquimolaren Menge in Bezug auf Kreatin eingebracht. Die Einführung von Derivaten von Aminosäuren und von kondensierendem Wirkstoff in die Lösung erfolgt nach der Auflösung des Kreatin- und p-TSS-Komplexes. Die Base wird 10 - 15 min nach dem Reaktionsbeginn und nach der Einstellung des Reaktionsgleichgewichts eingebracht, um das letztere in die vorgegebene Richtung zu verschieben.

Durch die Behandlung von Kreatin mit p-TSS werden gleichzeitig die Probleme der Verwandlung von Kreatin in eine Lösung und des Schutzes der Guanidin-Gruppe des Kreatins gelöst, wobei ein Komplex gebildet wird. Das erschwert die Umwandlung von Kreatin zum Kreatinin infolge der intramolekularen Ringbildung (Zyklisierung).

Als Kreatin werden wasserfreies Kreatin oder das billigere Kreatin-Monohydrat eingesetzt. Als kondensierender Wirkstoff können Carbodiimide, insbesondere Dicyctohexylcarbodiimid, N-Ethyl-N'-(3-Dimethylaminopropyl)carbodiimid, Diisopropylcarbodiimid oder Chlorameisensäuremonoester, insbesondere Chlorameisensäureethylester, Chlorameisensäureisobutylester, verwendet werden. Die Base bindet die sich abscheidende Salzsäure. Als Base werden die mit organischen Lösungsmitteln löslichen Basen verwendet, insbesondere tertiäre Amine, z. B. N-Methylmorpholin, Triäthylamin, Diisopropylethylamin usw. Als organische Lösungsmittel können Dimethylformamid, Dimethylacetamid,

Dimethylsulfoxid, N-Methylpyrrolidon usw. verwendet werden, die die vollständige Auflösung des Kreatin- und p-TSS-Komplexes sicherstellen.

Bevorzugt wird die Nutzung von Chlorameisensäuremonoestern, insbesondere Chlorameisensäureisobutylester oder Chlorameisensäureethylester als kondensierender Wirkstoff und Dicyclohexylcarbodiimid oder Diisopropylcarbodiimid als Carbodiimid, denn dabei wird die Menge von Fremdbeimengungen verringert, und das Verfahren der Aussonderung des Zielprodukts wird vereinfacht.

Die während des Vorgangs hergestellte Reaktionsmischung wird aufbereitet (gereinigt). Die Reinigung erfolgt in der Regel unter Einsatz von Ionenaustauschern mit nachfolgender Umkristallisation des Zielprodukts. Die Ausbeute des Zielprodukts erreicht 20%. Die Reinheit beträgt nach Angaben der HPLC-Chromatographie über 90%. Die Hauptbeimengung bei der Synthese ist Kreatinin. Der Kreatiningehalt im Zielprodukt beträgt in der Regel max. 1,5 %.

Die bei der Ausführung des vorliegenden Verfahrens hergestellten Kreatinamide können im Weiteren unter Einsatz der genormten Verfahren für die organische Synthese modifiziert werden.

Die Überwachung des Reaktionsablaufs sowie die Beurteilung der Reinheit der End- und Zwischenprodukte werden nach der Methode der HPLC-Chromatographie mit Phasenumkehr auf dem Chromatographen Alliance (Waters), Säulen Zorbax ODS, 3,5µm, 3 x 100 mm, (Agilent Technologies) vorgenommen. Zur Elution wird die Mischung einer Pufferlösung mit Acetonitril verwendet. Die Pufferlösung enthält 0,01 M Natriumoctansulfont und 0,02 M Natriumdihydrogenphosphat. Die Erkennung erfolgt nach der Methode der UV-Spektroskopie bei einer Wellenlänge von 210 nm.

Der Anteil des Hauptstoffs in den Kreatinamiden wird nach der Methode der Titration in nichtwässriger Lösung der Perchlorsäure im Eisessig in Anwesenheit des Erkennungsmittels Kristallviolett bestimmt.

Die Ermittlung der Molekularmasse der Kreatinamide wird nach dem Verfahren der Massenspektralanalyse mit Hilfe des Flugzeitmassenspektrometers MX-5303 mit einer Ionenquelle vom Typ "Elektrospray" (Filiale des Instituts für Energieprobleme der chemischen Physik der Russischen Akademie für Wissenschaften (Φ 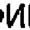 H∋ΠXΦ PAH)) ermittelt.

### Beispiel 1, Herstellung von Kreatinyl-glycin-isopropylesteracetat

Eine Suspension von 5,67 g (38 mM) Kreatin-Monohydrat in 40 ml Dimethylformamid wird in einen 250 ml großen Einhals-Rundkolben mit einem Tropftrichter mit Ausgleichsschleife gegeben. Der Tropftrichter ist dabei mit einem Kalziumchloridröhrchen geschlossen. Unter Rühren auf dem Magnetrührer werden 7,23 g (38 mM) p-Toluol-Sulfonsäure-Monohydrat nachgegeben. Nach etwa 5 min ist das Kreatin-Monohydrat völlig aufgelöst. Danach wird die Lösung mit 6,71 g (40 mM) Glycinisopropylester Hydrochlorid ergänzt. Nach seiner Auflösung wird die Reaktionsmischung bis zu einer Temperatur von -10° C im Wasser-Salz-Bad abgekühlt. Danach werden 5,24 ml (38 mM) Chlorameisensäurebutylester nachgesetzt. Im Laufe von 10 min werden 8,9 ml (81 mM) N-Methylmorpholin aus dem Tropftrichter dazugegeben. Die Reaktionsmischung wird 1 h lang im Eisbad vermengt. Danach wird die Temperatur langsam bis auf Raumtemperatur gebracht. 10 h später wird der ausgefallene Niederschlag von N- Methylmorpholin-Hydrochlorid ausgefiltert. Die Lösung wird im Vakuum bei einer Temperatur von 50° C eingedampft. Der ölartige Rückstand wird in 160 ml Chloroform aufgelöst und für 10 h bei einer Temperatur von -10° C stehen gelassen. Die Lösung wird ausfiltriert und mit Wasser (3 x 100 ml) extrahiert. Der gesammelte wässrige Auszug enthält das Zielprodukt. Dieser Auszug wird zweimal mit Chloroform extrahiert. Die Wasserlösung wird im Vakuum bis zu einer Menge von 50 ml eingedampft. Die hergestellte Lösung wird durch eine 30 x 250 mm große Säule im Wasser durchgelassen. Die Säule ist mit dem Sorptionsmittel Dowex 1 x 8 in Azetatform gefüllt. Der Eluent ist Wasser. Die Elutionsgeschwindigkeit beträgt 2 ml/min. Die Säule wird mit Wasser durchgespült. Dabei wird der pH-Wert des Eluats geprüft. Die Fraktionen mit einem pH-Wert von 7 (Wasser-pH=5) werden gesammelt, nach der Methode der HPLC-Chromatographie mit Phasenumkehr analysiert, vereinigt, mit Essigsäure ergänzt und eingedampft. Der Rückstand wird aus 20 ml Acetonitril bei einer Temperatur von -10° C innerhalb von 20 h kristallisiert. Der Absatz von Kreatinyl-glycin-isopropylesteracetat wird ausfiltriert, mit kaltem Acetonitril, Diethylether gespült und getrocknet.

Das hergestellte Produkt wird in 30 ml Äthylalkohol aufgelöst und 20 h lang bei einer Temperatur von -10° C gehalten. Die Lösung wird ausfiltriert. Äthylalkohol wird im Vakuum entfernt. Der Rückstand wird aus dem Diethylether kristallisiert.

Die Ausbeute von CpH₁₈N₄O₃·C₂H₄O₂ -Kreatinyl-glycin-isopropylesteracetat beträgt 2,3 g (21 %). Massenspektrum, gemessen: m/z: 290,29, berechnet: M 290,32. Der Gehalt an Kreatinyl-glycin-isopropylesteracetat gemäß Angaben der Titration in nichtwässriger Lösung beträgt 97,7% (Gew.); der Anteil der Kreatin-Beimischung beträgt 1,5% (Gew.).

### Beispiel 2, Herstellung von Kreatinyl-glycyl-glycin-ethylesteracetat

Eine Suspension von 4,77 g (32 mM) Kreatin-Monohydrat wird in 40 ml N-Methylpyrrolidon in einen 250 ml großen Einhals-Rundkolben mit einem Tropftrichter mit Ausgleichsschleife gegeben. Der Tropftrichter ist mit einem Kalziumchloridröhrchen geschlossen. Unter Umrühren werden 5,02 g (35 mM) wasserfreie p-Toluol-Sulfonsäure nachgesetzt. In etwa 5 min wird das Kreatin völlig aufgelöst. Danach werden 4,33 g (22 mM) Glycyl-glycinethylester-Hydrochlorid nachgesetzt.

Nach seiner Auflösung wird die Reaktionsmischung bis zu einer Temperatur von -10° C im Wasser-Salz-Bad gekühlt. Danach werden 4,51 ml (33 mM) von Chlorameisensäurebutylester zugegeben. Im Laufe von 10 min werden 5,1 ml (54mM) N-Methylmorpholin aus dem Tropftrichter zugegeben. Die Reaktionsmischung wird innerhalb von 1 h im Eisbad vermengt. Danach wird die Temperatur langsam bis auf Raumtemperatur gebracht. 10 h später wird der ausgefallene Niederschlag von N-Methylmorpholin-Hydrochlorid ausfiltriert. Das Lösemittel wird im Vakuum bei 50° C eingedampft. Der ölartige Rückstand wird in 100 ml Chloroform aufgelöst und für 10 h im Kühlschrank bei einer Temperatur von -10° C gelassen. Die Lösung wird ausfiltriert und mit Wasser extrahiert (3 x 100 ml). Der das Zielprodukt enthaltende gesammelte wässrige Auszug wird zweimal mit Chloroform extrahiert und im Vakuum bis zu einer Menge von 50 ml eingedampft.

Aussonderung des Zielprodukts nach dem Verfahren aus Beispiel 1 Die Ausbeute von C₁₀H₁₉N₅O₄·C₂H₄O₂ beträgt 1,05 g (14,6%). Massenspektrum, gemessen, beträgt m/z: 333,29, berechnet: M 333,29. Der Gehalt an Kreatinyl-glycyl-glycinisopropylesteracetat beträgt gemäß den Angaben der Titration in nichtwässriger Lösung 98,5% (Gew.), während der Anteil der Kreatinin-Beimischung 1,3% (Gew.) beträgt. Beispiel 3, Herstellung von Kreatinyl-glycinmethylester-Hydrochlorid Eine Suspension von 2,98 g (20 mM) Kreatin-Monohydrat in 20 ml Dimethylazetamid wird in einen Einhals-Rundkolben (250 ml) mit einem Tropftrichter mit Ausgleichsschleife gegeben. Der Tropftrichter ist mit einem Kalziumchloridröhrchen geschlossen. Unter Rühren auf einem Magnetrührer werden 3,99 g (21 mM) von p-Toluol-Sulfonsäure- Monohydrat nachgesetzt. In etwa 5 min wird der Absatz völlig aufgelöst. Danach wird die Mischung mit 2,64 g (21 mM) Glycinmethylester-Hydrochlorid ergänzt. Nach seiner Auflösung wird die Reaktionsmischung bis zu einer Temperatur von -10° C im Wasser-Salz-Bad abgekühlt. Danach werden 2,8 ml (20 mM) von Chlorameisensäureisobutylester eingebracht. Im Laufe von 10 min werden 4,4 ml (40 mM) N-Methylmorpholin aus dem Tropftrichter zugegeben. Die Reaktionsmischung wird innerhalb von 1 h im Eisbad vermengt. Danach wird die Temperatur langsam bis auf Raumtemperatur gebracht. Nach 7 h wird der ausgefallene Niederschlag von N-Methylmorpholin-Hydrochlorid ausfiltriert. Das Produkt wird mit Hexan abgesetzt. Der ölartige Absatz wird abgetrennt. Der Absatz wird in 80 ml Chloroform aufgelöst und für 10 h im Kühlschrank bei einer Temperatur von -10° C stehen gelassen. Die Lösung wird ausfiltriert und mit Wasser extrahiert (3 x 100 ml). Der gesammelte das Zielprodukt enthaltende wässrige Auszug wird mit Chloroform extrahiert (2 x 80 ml) und im Vakuum bis zu einer Menge von 50 ml eingedampft. Die Reinigung des Zielprodukts erfolgt unter den Bedingungen des Beispiels 1 mit Ausnahme davon, dass die Fraktionen mit einem pH-Wert von 7 (Wasser- pH= 5) gesammelt, nach der Methode der HPLC-Chromatographie mit Phasenumkehr analysiert, vereinigt, mit Salzsäure ergänzt und eingedampft werden.

Die Ausbeute von C₇H₁₄N₄O₃·HCl beläuft sich auf 1,52 g (30%). Das Massenspektrum beträgt, gemessen: m/z: 238,65, berechnet: M 238,67. Der Gehalt an Kreatinylglycinmethylester-Hydrochlorid beträgt gemäß den Angaben der Titration in nichtwässriger Lösung 98,3% (Gew.), während der Anteil der Kreatinin-Beimischung 1,5 % (Gew.) beträgt.

### Beispiel 4, Herstellung von Kreatinyl-glyclnethylamidtartrat aus wasserfreiem Kreatin

Eine Suspension von 2,62 g (20 mM) Kreatin in 20 ml Dimethylformamid wird in einen Einhals-Rundkolben (250 ml) mit einem Tropftrichter mit Ausgleichsschleife gegeben. Der Tropftrichter ist mit einem Kalziumchloridröhrchen geschlossen. Die Mischung wird unter Rühren auf einem Magnetrührer mit 3,83 g (20,11 mM) p-Toluol-Sulfonsäure-Monohydrat ergänzt. Der Absatz löst sich in etwa10 min völlig auf. Danach werden 4,77 g (22 mM) Glycinethylamid-Trifluoracetat zugegeben. Nach seiner Auflösung wird die Reaktionsmischung bis zu einer Temperatur von -10° C im Wasser-Salz-Bad abgekühlt. Danach werden 2,8 ml (20 mM) Chlorameisensäureisobutylester eingebracht. Im Laufe von 10 min werden 4,7 ml (43 mM) N-Methylmorpholin aus dem Tropftrichter zugegeben. Die Reaktionsmischung wird 1 h lang im Eisbad vermengt. Danach wird die Temperatur langsam bis auf Raumtemperatur gebracht. Nach 20 h wird der ausgefallene Niederschlag ausfiltriert. Das Lösungsmittel wird im Vakuum bei 50° C eingedampft. Der ölartige Rückstand wird in 80 ml Chloroform gelöst und für 20 h im Kühlschrank bei einer Temperatur von -10° C stehen gelassen. Die Lösung wird ausfiltriert und mit Wasser extrahiert (3 x 100 ml). Der gesammelte wässrige Auszug mit dem Zielprodukt wird mit Chloroform extrahiert (2 x 80 ml) und wird im Vakuum bis zu einer Menge von 50 ml eingedampft. Das hergestellte Produkt wird nach dem aus Beispiel 1 bekannten Verfahren ausgesondert mit Ausnahme davon, dass eine mit dem Sorptionsmittel Dowex 1 x 8 in der Tartratform gefüllte Säule eingesetzt wird. Die das Zielprodukt enthaltenden Fraktionen werden nach der Methode der HPLC-Chromatographie mit Phasenumkehr analysiert, vereinigt und eingedampft.

Die Ausbeute von [C₈H₁₇N₅O₂]₂·C₄H₆O₆ beträgt 3,7 g (32%). Das Massenspektrum beträgt, gemessen: m/z: 583,62, berechnet: M 583,61. Der Gehalt an der Hauptsubstanz Kreatinyl-glycin-ethylamidtartrat beträgt gemäß den Angaben der Titration in nichtwässriger Lösung 98,9% (Gew.), während der Anteil der Kreatinin-Beimischung 0,7 % (Gew.) beträgt.

### Beispiel 5, Herstellung von N^{α}-Carbobenzoxy-N^{ε}-Kreatinyl-lysin-ethylesterdiacetat

Eine Suspension von 3 g (20,5 mM) Kreatin-Monohydrat in 40 ml Dimethylformamid wird in einen 250 ml großen Einhals-Rundkolben mit einem Tropftrichter mit Ausgleichsschleife gegeben. Der Tropftrichter wird mit einem Kalziumchloridröhrchen geschlossen. 3,81 g (20,5 mM) p-Toluol-Sulfonsäure-Monohydrat werden unter Umrührern dazu gegeben. 10 min später werden 7 g (20,5 mM) α-Carbobenzoxy-L-lysinethylester-Hydrochlorid nachgesetzt. Nach seiner Auflösung wird die Reaktionsmischung bis zu einer Temperatur von -10° C im Wasser-Salz-Bad gekühlt. Danach werden 2,8 ml (20,5 mM) Chlorameisensäureisobutylester eingeführt. Im Laufe von 10 min werden 4,4 ml (20.5 mM) N-Methylmorpholin aus dem Tropftrichter zugegeben. Die Reaktionsmischung wird 1 h lang im Eisbad vermengt. Danach wird die Temperatur langsam bis auf Raumtemperatur gebracht. 10 h später wird die Suspension gefiltert. Das Lösungsmittel wird im Vakuum bei einer Temperatur von 50° C eingedampft. Der ölartige Rückstand wird in 150 ml n-Butanol aufgelöst, viermal mit 5%iger Lösung von NaHCO₃ und zweimal mit Wasser gespült. Anschließend wird das n-Butanol eingedampft. Der Rückstand wird mit 30 ml Wasser ergänzt. Die hergestellte Suspension wird auf eine Säule mit dem Sorptionsmittel YMC*Gel ODS 22.5 x 150, ausgeglichen in 0,2%iger Essigsäure, aufgetragen. Die Säule wird mit der 0,2%igen Lösung von Essigsäure gespült. Danach erfolgt die Elution mit einem Lineargradienten bis zu 10% von Isopropylalkohol in der 0,2%igen Essigsäure-Lösung. Die Fraktionen werden mit Hilfe von guten Reaktionen und der HPLC-Chromatographie analysiert, vereinigt und eingedampft. Der Rückstand wird aus 5 ml Azetonitril kristallisiert, mittels Filterung abgetrennt und im Vakuum getrocknet.

Die Ausbeute von C₂ₒH₃₁N₅O₅·C₂H₄O₂- N^{α}-Carbobenzoxy-N^{ε}-kreatinyl-lysinethylesteracetat beträgt 1,5 g (18%). Das Massenspektrum beträgt, gemessen: m/z: 481,77, berechnet: M 481,77. Der Gehalt an Hauptsubstanz N^{α}-Karbobenzoxy-N^{ε}-Kreatinyl-lysinethylestheracetat beträgt gemäß den Angaben der Titration in nichtwässriger Lösung 98,9% (Gew.), während der Anteil der Kreatinin-Beimischung 1,0 % (Gew.) beträgt.

1,0 g des hergestellten N^{α}-Carbobenzoxy-N^{ε}-Kreatinyl-lysin-ethylesteracetat wird in 10 ml Methylalkohol gelöst und über Palladiumschwarz innerhalb von 3 h hydriert. Die vollständige Entfernung der Carbobenzoxygruppe wird mit Hilfe der Dünnschichtchromatographie in einem System Azetonitril:Wasser:Essigsäure im Verhältnis 6:1:1 geprüft. Der Katalysator wird ausfiltriert. Das Filtrat wird mit 1 ml Essigsäure ergänzt und eingedampft. Der Rückstand wird aus 10 ml Acetonitril kristallisiert. Das Produkt wird ausfiltriert, mit kaltem Acetonitril-Äther gespült und im Vakuum getrocknet.

Die Ausbeute an C₁₀H₂₁N₅O₃·2C₂H₄O₂- N^{ε}-Kreatinyl-lysin-ethylesterdiacetat beläuft sich auf 0,4 g (84%). Das Massenspektrum beträgt, gemessen: m/z: 379,41,berechnet: M 379,41. Der Gehalt an der Hauptsubstanz N^{ε}-Kreatinyllysinethylesterdiacetat beträgt gemäß den Angaben der Titration in nichtwässriger Lösung 99,2% (Gew.), während der Anteil der Kreatinin-Beimischung 0,5% (Gew.) beträgt.

### Beispiel 6, Herstellung von Kreatlnyl-phenyl-alanin-ethylamid-Hydrochlorid aus wasserfreiem Kreatin.

Eine Suspension von 2,62 g (20 mM) Kreatin in 20 ml Dimethylsulfoxid wird in einen 250 ml großen Einhals-Rundkolben mit einem Tropftrichter mit Ausgleichsschleife gegeben. Der Tropftrichter wird mit einem Kalziumchloridröhrchen geschlossen. 3,83g (20,11 mM) p-Toluol-Sulfonsäure-Monohydrat wird unter Rühren auf einem Magnetrührer nachgesetzt. In etwa 10 min wird der Niederschlag völlig aufgelöst. Danach werden 6,71 g (20 mM) Phenylalaninethylamid-Trifluoracetat nachgesetzt.

Nach seiner Auflösung wird die Reaktionsmischung bis zu einer Temperatur von -10° C im Wasser-Salz-Bad vermengt. Danach werden 2,8 ml (20 mM) Chlorameisensäureisobutylester eingeführt. Im Laufe von 10 min werden 4,65 ml (43 mM) Morpholin aus dem Tropftrichter dazu gegeben. Die Reaktionsmischung wird 1 h lang im Eisbad vermengt. Danach wird die Temperatur langsam bis auf Raumtemperatur gebracht. Nach 20 h wird der ausgefallene Niederschlag ausfiltriert. Das Lösemittel wird im Vakuum bei 50°C eingedampft. Der ölartige Rückstand wird in 80 ml Chloroform aufgelöst und für 20 h bei einer Temperatur von -10°C stehen gelassen. Die Lösung wird ausfiltriert und mit Wasser extrahiert (3 x 100 ml). Der gesammelte das Zielprodukt enthaltende wässrige Auszug wird mit Chloroform (2 x 80 ml) extrahiert und im Vakuum bis einer Menge von 50 ml eingedampft. Das hergestellte Produkt wird nach dem aus Beispiel 1 bekannten Verfahren ausgesondert mit Ausnahme davon, dass die Fraktionen mit einem pH-Wert von 7 (Wasser-pH=5) gesammelt werden, nach der Methode der HPLC-Chromatographie mit Phasenumkehr analysiert, vereinigt, mit 1 M Salzsäurelösung ergänzt und eingedampft.

Die Ausbeute an C₁₅H₂₃N₅O₂·HCl-Kreatinyl-phenylalanin-ethylamid-Hydrochlorid beträgt 3,7 g (32%). Das Massenspektrum beträgt, gemessen: m/z: 341,87, berechnet: M 341,84. Der Gehalt an der Hauptsubstanz Kreatinyl-phenylalanin-ethylamid-Hydrochlorid beträgt gemäß den Angaben der Titration in nichtwässriger Lösung 99,3% (Gew.), während der Anteil der Kreatinin-Beimischung 0,5 % (Gew.) beträgt.

### Beispiel 7, Herstellung von Kreatinyl-glycyl-alaninethylester-Hemisuccinat

Eine Suspension von 2 g (13,4 mM) Kreatin-Monohydrat in 10 ml Dimethylformamid wird in einen 100 ml großen Einhals-Rundkolben mit einem Tropftrichter mit Ausgleichsschleife gegeben. Der Tropftrichter wird mit einem Kalziumchloridröhrchen geschlossen. Unter Rühren auf einem Magnetrührer werden zuerst 2,54 g (13,4 mM) p-Toluol-Sulfonsäure-Monohydrat und anschließend 1,41 g (6,7 mM) Glycylalaninethylester-Hydrochlorid und 1,38 g (6,7 mM) Dicyclohexylcarbodiimid in 2 ml Dimethylformamid zugegeben. Dann werden 1,14 ml (6,7 mM) Diisopropyläthylamin aus dem Tropftrichter im Laufe von 10 min zugegeben. Die Reaktionsmischung wird für 20 h bei Raumtemperatur stehen gelassen. Der Niederschlag von Diisopropyläthylamin-Hydrochloriden und Dicyclohexylharnstoff wird ausfiltriert. Die Mutterlösung wird bei einer Temperatur von 50° C eingedampft. Der ölartige Rückstand wird in 100 ml Chloroform gelöst und mit Wasser extrahiert (3 x 100 ml). Der gesammelte das Zielprodukt enthaltende wässrige Auszug wird zweimal mit Chloroform extrahiert und in einem Rotorverdampfer bis auf einen Umfang von 20 ml eingedampft. Die hergestellte Lösung wird durch eine 30 x 150 mm große mit Dowex 2 x 8 in Form von Succinat gefüllte Säule durchgelassen und mit Hilfe von Wasser eluiert (ausgewaschen). Die Elutionsgeschwindigkeit beträgt 2 ml/min. Die Säule wird mit Wasser durchgespült. Dabei wird der pH-Wert des Eluats geprüft. Die Fraktionen mit einem pH-Wert von 6 - 7 werden gesammelt, vereinigt und eingedampft. Der Rückstand wird aus 10 ml Acetonitril bei einer Temperatur von -10° C im Laufe von 5 h kristallisiert. Der Absatz von Kreatinyl-glycyl-alaninethylester-Hemisuccinats wird ausfiltriert, mit kaltem Acetonitril und Diethylether gespült und getrocknet. Die Rohausbeute beträgt 1,8 g.

Das Produkt wird in 15 ml Äthylalkohol gelöst und 10 h lang bei einer Temperatur von -10° C gehalten. Die Lösung wird filtriert. Die Mutterlösung wird eingedampft. Der Rückstand wird in 5 ml Wasser aufgelöst und auf eine Säule mit YMO*Gel ODS 22.5 x 150 aufgetragen. Dabei wird die Säule in 0,05%iger Bernsteinsäure im Wasser ausgeglichen. Die Elution wird mit Hilfe von 0,02%iger Bernsteinsäure vorgenommen. Die Fraktionen werden anhand der HPLC-Chromatographie ausgewertet. Die das Zielprodukt enthaltenden Fraktionen werden vereinigt. Die Reinheit des Zielprodukts beträgt mindestens 95%. Die vereinigten Fraktionen werden eingedampft. Der Rückstand wird mittels Destillation (Entgeistung) mit Iso-Propylalkohol getrocknet und aus dem Diethylether kristallisiert.

Die Ausbeute an C₁₁H₂₁N₅O₄·0,5C₄H₆O₄-Kreatinyl-glycin-alanin-ethylester-Hemisuccinats beträgt 0,56 g (24%). Das Massenspektrum beträgt, gemessen: m/z: 346,33, berechnet: M 346,36. Der Gehalt an der Hauptsubstanz Kreatinyl-glycyl-alaninethylester-Hemisuccinats beträgt gemäß den Angaben der Titration in nichtwässriger Lösung 99,4% (Gew.). Der Anteil der Kreatinin-Beimischung beträgt laut der HPLC-Chromatographie 0,8% (Gew.).

### Beispiel 8, Herstellung von Kreatinil-γ-Aminobuttersäure aus Äthylätherazetat

Eine Suspension von 4,20 g (32 mM) Kreatin in 40 ml Dimethylformamid wird in einen 250 ml großen Einhals-Rundkolben mit einem Tropftrichter mit Ausgleichsschleife gegeben. Der Tropftrichter wird mit einem Kalziumchloridröhrchen geschlossen. Unter Umrühren wird diese Mischung mit 5,02 g (35 mM) p-Toluol-Sulfonsäure und 5,36 g (32 mM) γ-Aminobuttersäureethylester-Hydrochlorid ergänzt. Nach seiner Auflösung wird die Reaktionsmischung bis zu einer Temperatur von -10° C im Wasser-Salz-Bad abgekühlt. Danach wird das Gemisch mit 3,2 ml (33 mM) Chlorameisensäureethylester ergänzt. Im Laufe von 10 min werden 5,1 ml (54 mM) N-Methylmorpholin aus dem Tropftrichter zugegeben. Die Reaktionsmischung wird 1 h lang im Eisbad vermengt. Danach wird die Temperatur langsam bis auf Raumtemperatur gebracht. 10 h später wird der Absatz von N-Methylmorpholin-Hydrochlorid ausfiltriert. Die Mutterlösung wird im Vakuum bei 50° C eingedampft. Der Rückstand wird aus dem Isopropylalkohol umkristallisiert. Anschließend wird der Rückstand mit Hilfe der Austauschchromatographie gereinigt. Die Reinigung erfolgt auf der mit Sephadex SE C25 gefüllten Säule im Pyrdin-Acetat-Puffer.

Die das Zielprodukt enthaltenden Fraktionen werden vereinigt und eingedampft.

Die Ausbeute an C₁₀H₂₀N₄O₃·C₂H₄O₂-Kreatinil-γ-Aminobuttersäure-Äthylätherazetat beträgt 1,95 g (25,0%). Das Massenspektrum beträgt, gemessen m/z: 304,34, berechnet: M 304,35. Der Gehalt an der Hauptsubstanz Kreatinyl-γ-aminobuttersäureethylesteracetat beträgt gemäß den Angaben der Titration in nichtwässriger Lösung 98,7% (Gew.). Der Anteil der Kreatin-Beimischung beträgt gemäß den Angaben der HPLC-Chromatographie 1,2% (Gew.).

### Beispiel 9, Herstellung von Kreatinyl-alanin-ethylesteracetat

Eine Suspension von 2 g (13,4 mM) Kreatin-Monohydrat in 10 ml Dimethylformamid wird in einen 100 ml großen Einhals-Rundkolben mit einem Tropftrichter mit Ausgleichsschleife gegeben. Der Tropftrichter wird mit einem Kalziumchloridröhrchen geschlossen. Unter Rühren auf einem Magnetrührer werden 2,54 g (13,4 mM) p-Toluol-Sulfonsäure-Monohydrat und anschließend 1,01 g (6,7 mM) Alaninethylester-Hydrochlorid und 0,85 g (6,7 mM) Diisopropylcarbodiimid in 2 ml Dimethylformamid nachgesetzt. Danach werden 0,94 ml (6,7 mM) Triethylamin im Laufe von 10 min aus dem Tropftrichter zugegeben. Die Reaktionsmischung wird für 20 h bei Raumtemperatur stehen gelassen. Der ausgefallene Niederschlag der Hydrochloride des Diisopropylethylamins und Diisopropyl-Harstoffs wird ausfiltriert. Die Mutterlösung wird bei einer Temperatur von 50° C eingedampft. Der ölartige Rückstand wird in 100 ml Chloroform aufgelöst und mit Wasser extrahiert (3 x 100 ml). Der das Zielprodukt enthaltende gesammelte wässrige Auszug wird zweimal mit Chloroform extrahiert und in einem Rotorverdampfer bis zu einem Umfang von 20 ml eingedampft.

Die hergestellte Lösung wird durch eine 30 x 150 mm große, mit Dowex 2 x 8 in der Acetatform-Säule durchgelassen und mit Wasser eluiert. Die Elutionsgeschwindigkeit beträgt 2 ml/min. Die Säule wird mit Wasser durchgespült. Dabei wird der pH-Wert des Eluats geprüft. Die Fraktionen mit einem pH-Wert von 6 - 7 werden gesammelt, vereinigt und eingedampft. Der Rückstand wird aus 10 ml Acetonitril bei einer Temperatur von -10° C im Laufe von 5 h kristallisiert. Der Niederschlag des Kreatinyl-alaninethylesteracetats wird ausfiltriert, mit Kaltem Acetonitril und Diethylether gespült und getrocknet.

Die Ausbeute an CgH₁₈N₄O₃·C₂H₄O₂-Kreatinyl-alanin-ethylesteracetat beträgt 0,56 g (24%). Das Massenspektrum beträgt, gemessen m/z: 290,35, berechnet: M 290,31. Der Gehalt an der Hauptsubstanz Kreatinyl-alanin-ethylesteracetat beläuft sich gemäß den Angaben der Titration in nichtwässriger Lösung auf 99,1 % (Gew.). Der Anteil der Kreatinin-Beimischung beträgt laut den Angaben der HPLC-Chromatographie 0,9% (Gew.).

### Beispiel 10, Herstellung von Kreatinyl-phenylalanin-ethylesteracetat

Eine Suspension von 11,3 g (86 mM) Kreatin in 80 ml Dimethylformamid wird in einen 250 ml großen Einhals-Rundkolben mit einem Tropftrichter mit Ausgleichsschleife gegeben. Der Tropftrichter wird mit einem Kalziumchloridröhrchen geschlossen. 16 g (86 mM) p-Toluol-Sulfonsäure-Monohydrat wird unter Rühren auf einem Magnetrührer nachgesetzt. Der Absatz löst sich in etwa 5 min völlig auf. Danach wird die Mischung mit 11,5 g (50 mM) Pheylalaninethylester-Hydrochlorid und 7,75 ml (45 mM) Diisopropylkarbodiimid ergänzt. Dann werden 8,6 ml (50 mM) Diisopropylethylamin aus dem Tropftrichter im Laufe von 10 min zugegeben. Die Reaktionsmischung wird für 20 h bei Raumtemperatur stehen gelassen und dann mit 10 ml Wasser ergänzt. Der ausgefallene Niederschlag des Diisopropyl-Harnstoffs wird ausfiltriert. Die resultierende Lösung wird mit Hilfe der HPLC-Chromatographie analysiert. Nach Angaben der Analyse betrug der Wirkungsgrad der Reaktion 50%.

Das hergestellte Produkt wird nach dem aus Beispiel 1 bekannten Verfahren ausgesondert mit Ausnahme davon, dass die Fraktionen mit einem pH-Wert von 7 (Wasser-pH=5) gesammelt, nach der Methode der HPLC-Chromatographie mit Phasenumkehr analysiert, vereinigt, mit 1 M Essigsäure-Lösung ergänzt und eingedampft werden.

Die Ausbeute an C₁₅H₂₂N₄O₃·C₂H₄O₂-Kreatinyl-phenylalanin-ethylesteracetat beträgt 7,9 g (25%). Das Massenspektrum beträgt, gemessen m/z: 366,37, berechnet: M 366,41. Der Gehalt an der Hauptsubstanz Kreatinyl-phenylalanin-ethylesteracetat beläuft sich gemäß den Angaben der Titration in nichtwässriger Lösung auf 99,4% (Gew.). Der Anteil der Kreatinin-Beimischung beträgt 0,7% (Gew.).
Studium der neuroprotektiven Aktivität der Kreatinamide

Die Untersuchungen sind hier und nachfolgend unter Anwendung von folgenden nach dem vorliegenden Verfahren hergestellten Kreatinamiden durchgerührt worden:
1. Kreatinyl-glycin-isopropylesteracetat
2. Kreatinyl-glycyl-glycin-ethylesteracetat
3. Kreatinyl-glycin-ethylamidtartrat
4.N^{α}-Carboxybenzoxy-N^{ε}-Kreatinyl-lysin-ethylesteracetat
5. Kreatinyl-phenylalanin-ethylamid-Hydrochlorid
6. Kreatinyl-γ-aminobuttersäure-ethylesteracetat

Die neuroprotektive Wirkung von Kreatinamiden wurde an Modellen der Herdischämie bei männlichen Ratten Vistar, Alter 12 - 14 Wochen, Körpergewicht 220 - 240 g, erforscht. Zur Anästhesie wurde Pentothal-Natrium (Trapanalnarkose) in einer Dosis von 60 mg/kg verwendet. Die Tiere aus der 1. Gruppe bekamen die Lösung von Kreatinamid in der physiologischen Lösung intravenös 45 Minuten vor der Ischämie. Der Referenzgruppe wurde die physiologische Lösung verabreicht. Die zweite Gruppe von Tieren bekam die Lösung Kreatinamid in der physiologischen Lösung 24 Stunden vor der Ischämie dreimal am Tag ventrikulär (*per* os) mittels einer Sonde eingeführt. Die Referenzgruppe bekam die physiologische Lösung verabreicht.

Die endovaskuläre Okklusion der mittleren Hirnschlagader wurde nach der Methode von Koizumi, J., et al. (1986), unter Berücksichtigung von Modifikationen von Longa, E.Z. et al. (1989) und Belayev L. et al. (1999) vorgenommen. Die Normalfristen der Ischämie betrugen 39 Minuten, und die Reperfusion betrug 48 Stunden. Um das Ausmaß der Hirnschädigung der Tiere einzuschätzen, wurden die Tiere abgetötet.

Es wurden 2 mm starke Koronarschnitte vorbereitet. Die Schädigungsfläche wurde mit Hilfe einer Anfärbung von Phenyltetrazolchlorid bestimmt. Anschließend wurden die digitalen Aufnahmen der Schädigung vorgenommen. Der gefärbte aufgenommene Schnitt wurde ausgewertet. Dabei wurde der Gehirnschädigungsfaktor berechnet: Verhältnis der Schädigungsfläche zur gesamten Schnittoberfläche (Tabelle 1).

**Tabelle 1, Einwirkung von Kreatinamiden auf die Hirnschädigung der männlichen Ratten Vistar bei experimenteller Ischämie/Reperfusion (p<0,05)**

| Präparat | Art der Verabreichung, Dosis (n = 5) | Gehirnschädigungsfaktor [%] |
|---|---|---|
| Präparat 1 | intravenös; 150mg/kg | 11,2±2,3 |
| Präparat 2 | intravenös; 200mg/kg | 12,1±2,0 |
| Präparat 3 | intravenös; 100mg/kg | 12,0±1,7 |
| Präparat 4 | intravenös; 100mg/kg | 11,3±3,2 |
| Präparat 5 | intravenös; 50mg/kg | 14,0±1,1 |
| Präparat 6 | intravenös; 45mg/kg | 13,1±1,0 |
| Physiologische Lösung | intravenös | 19,4±5,3 |
| Präparat 1 | *per os*; 3x150mg/kg | 14,2±3,0 |
| Präparat 3 | *per os*; 3x150mg/kg | 13,9±3,3 |
| Präparat 4 | *per os*; 3x100mg/kg | 15,2±2,9 |
| Physiologische Lösung | *per os* | 21,4±4,7 |

Forschung des Einflusses von Kreatinamiden auf die Erhaltung der kognitiven Funktionen bei Gehirnischämie.

Die Gehirnischämie wurde nach der oben beschriebenen Methode reproduziert. Die Lösung von Kreatinamid oder die physiologische Lösung wurden den Tieren intravenös verabreicht. Um den neurologischen Mangel bei der Ischämie- und Reperfusions-Gehirnherdschädigung einzuschätzen, wurde die Skala von Garcia et al. (1955) benutzt. Die Tiere wurden vor der Ischämie, am zweiten und am dritten Tag nach der Ischämie/Reperfusion um eine festgelegte Zeit getestet, um die Verhaltensänderungen durch den Zirkadianrhythmus auszuschließen. Folgendes wurde eingeschätzt: spontane Aktivität, Symmetrie bei der Bewegung von Extremitäten, Besteigen einer senkrechten Gitterwand, propriozeptive Sensibilität des Körpers, Reaktionen beim Berühren der Schnurrhaare. Die max. Punktezahl gemäß dieser Skala ist 18 (kein neurologischer Mangel liegt vor), die minimale Punktezahl ist 3 (schwerer neurologischer Mangel liegt vor). Die Ergebnisse sind in Tabelle 2 angeführt.

**Tabelle 2, Nervenzustand der Ratten nach der Garcia-Skala am 2. und 3. Tag nach der Ischämie (p<0,05)**

| Präparat, Dosis | Punkte, Nervenzustand nach der Garcia-Skala (n=5) | | |
|---|---|---|---|
| | 24 Stunden vor der Ischämie | 2. Tag nach der Ischämie | 3. Tag nach der Ischämie |
| Präparat 1; 150 mg/kg | 18±0 | 10±2 | 15±2 |
| Präparat 3; 100 mg/kg | 18±0 | 11±2 | 16±1 |
| Präparat 4; 100 mg/kg | 18±0 | 14±1 | 17±2 |
| Präparat 5; 50 mg/kg | 18±0 | 11±1 | 14±1 |
| Präparat 6; 45 mg/kg | 18±0 | 14±0 | 16,0±1,1 |
| Physiologische Lösung | 18±0 | 6±1 | 9±2 |

Forschung von Haltbarkeit der Kreatinamide im künstlichen Magensaft und Blutplasma des Menschen

Um die Haltbarkeit der Kreatinamide zu erforschen, wurden 10 mg Kreatinamid in 20 ml künstlichem Magensaft aufgelöst. Die aliquote Lösung wurde in ein Fläschchen für den Chromatographen gesetzt. Das Fläschchen mit der Lösung wurde in die Probenahmevorrichtung des Chromatographen bei einer Temperatur von 37° C gesetzt. Mit Hilfe der HPLC-Chromatographie wurden die Anfangskonzentration des Kreatinamids und ihre relative Veränderung im Magensaft bei einer vorgegebenen Temperatur ermittelt (Tabelle 3).

**Tabelle 3, Haltbarkeit der Kreatinamide im künstlichen Magensaft bei einer Temperatur von 37° C**

| Präparat | Haltbarkeit [%] | | | |
|---|---|---|---|---|
| | 0 St. | 1 St. | 3 St. | 5 St. |
| Präparat 1 | 100 | 100 | 99 | 97 |
| Präparat 2 | 100 | 100 | 98 | 98 |
| Präparat 3 | 100 | 100 | 97 | 98 |
| Präparat 4 | 100 | 100 | 98 | 97 |
| Präparat 5 | 100 | 100 | 98 | 99 |
| Präparat 6 | 100 | 100 | 99 | 99 |

Die Haltbarkeit der Kreatinamide im Blutplasma des Menschen wurde anhand der relativen Veränderung der Konzentration des Kreatinamids im Plasma eingeschätzt. 1 ml Plasma wurde mit 0,2 ml Wasserlösung des Kreatinamids in einer Konzentration von 5 mg/ml vermengt. Das Gemisch wurde bei einer Temperatur von 37° C gehalten. Die Aliquote in einer Menge von 0,2 ml wurde in gleichen Zeitabständen entnommen und mit 0,02 ml der 10%igen Lösung der Trichloressigsäure vermischt. Der Niederschlag wurde 15 min später bei 300 g im Laufe von 20 min zentrifugiert. Die Konzentration des Kreatinamids im Supernatant wurde nach der Methode der HPLC-Chromatographie bestimmt (Tabelle 4).

**Tabelle 4, Haltbarkeit der Kreatinamide im Blutplasma des Menschen bei einer Temperatur von 37° C**

| Präparat | Haltbarkeit [%] | | | |
|---|---|---|---|---|
| | 0 St. | 0,25 St. | 0,5 St. | 1 St. |
| Präparat 1 | 100 | 100 | 99 | 91 |
| Präparat 2 | 100 | 100 | 97 | 90 |
| Präparat 3 | 100 | 100 | 95 | 90 |
| Präparat 4 | 100 | 100 | 100 | 89 |
| Präparat 5 | 100 | 100 | 97 | 87 |

Die angeführten Ergebnisse haben gezeigt, dass es beim Einsatz des Verfahrens gemäß der Erfindung gelingt, Kreatinamide nach einer einfacheren Verfahrenstechnik, aus billigerem Rohmaterial und mit höherer Ausbeute herzustellen. Die hergestellten Kreatinamide sind für die Anwendung in der Medizin von Interesse, denn sie weisen eine neuroprotektive Wirkung auf.

## Patentansprüche

1. Verfahren zur Herstellung von Kreatinamiden in Form von Salzen,
**gekennzeichnet durch** folgende Verfahrensschritte:
- Behandlung von Kreatin mit p-Toluol-Sulfonsäure im organischen Lösemittel,
- nachfolgende Zusammenwirkung des hergestellten Komplexes mit Derivaten der Aminosäuren, die eine primäre bzw. sekundäre Aminogruppe enthalten, unter Anwesenheit eines konsequent eingebrachten kondensierenden Wirkstoffs, vorzugsweise Carbodiimide oder Chlorameisensäureester, und einer Base, vorzugsweise tertiäre Amine.

2. Verfahren nach Anspruch 1,
**dadurch gekennzeichnet,**
**dass** die Behandlung mit der p-Toluol-Sulfonsäure mindestens in einer äquimolaren Menge in Bezug auf Kreatin erfolgt.

3. Verfahren nach Anspruch 1,
**dadurch gekennzeichnet,**
**dass** als Kreatin wasserfreies Kreatin verwendet wird.

4. Verfahren nach Anspruch 1,
**dadurch gekennzeichnet,**
**dass** als Kreatin Kreatin-Monohydrat verwendet wird.

5. Verfahren nach Anspruch 1,
**dadurch gekennzeichnet,**
**dass** Ester- oder Amid-Derivate von aliphatischen oder aromatischen Aminosäuren als Derivate von Aminosäuren verwendet werden.

6. Verfahren nach Anspruch 1,
**dadurch gekennzeichnet,**
**dass** Dicyclohexylcarbodiimid als kondensierender Wirkstoff verwendet wird.

7. Verfahren nach Anspruch 1,
**dadurch gekennzeichnet,**
**dass** Diisopropyl-Carbodiimid als kondensierender Wirkstoff verwendet wird.

8. Verfahren nach Anspruch 1,
**dadurch gekennzeichnet,**
**dass** Chlorameisensäureisabutylester als kondensierender Wirkstoff verwendet wird.

9. Verfahren nach Anspruch 1,
**dadurch gekennzeichnet,**
**dass** Chlorameisensäureethylester als kondensierender Wirkstoff verwendet wird.

10. Verfahren nach Anspruch 1,
**dadurch gekennzeichnet,**
**dass** als Base die mit organischen Lösungsmitteln lösliche Basen verwendet werden und
**dass** ihre Mengen die Bindung der sich abscheidenden Säure sicherstellen.

11. Verfahren nach Anspruch 1,
**dadurch gekennzeichnet,**
**dass** als Base N-Methylmorpholin verwendet wird.

12. Verfahren nach Anspruch 1,
**dadurch gekennzeichnet,**
**dass** als Base Triäthylamin verwendet wird.

13. Verfahren nach Anspruch 1,
**dadurch gekennzeichnet,**
**dass** als Base Diisopropyläthylamin verwendet wird.

14. Verfahren nach Anspruch 1,
**dadurch gekennzeichnet,**
**dass** als organisches Lösemittel Dimethylacetamid, N-Methylpyrrolidon, Dimethylsulfoxid oder Dimethylformamid verwendet werden.

## Claims

1. Method for the preparation of creatine amides in the form of salts, **characterised by** the following method steps:
- treatment of creatine with p-toluene sulphonic acid in an organic solvent,
- subsequent interaction of the complex produced with derivatives of amino acids containing a primary or secondary amino group, in the presence of a consistently introduced condensing agent preferably carbodiimide or chloroformate, and a base, preferably tertiary amines.

2. Method according to claim 1, **characterised in that** the treatment with the p-toluene sulphonic acid is carried out at least in an equimolar amount with respect to creatine.

3. Method according to claim 1, **characterised in that** anhydrous creatine is used as a creatine.

4. Method according to claim 1, **characterised in that** monohydrate creatine is used as a creatine.

5. Method according to claim 1, **characterised in that** ester or amide derivatives of aliphatic or aromatic amino acids are used as derivatives of amino acids.

6. Method according to claim 1, **characterised in that** dicyclohexylcarbodiimide is used as the condensing agent.

7. Method according to claim 1, **characterised in that** diisopropyl-carbodiimide is used as the condensing agent.

8. Method according to claim 1, **characterised in that** isobutyl chloroformate is used as the condensing agent.

9. Method according to claim 1, **characterised in that** ethyl chloroformate is used as the condensing agent.

10. Method according to claim 1, **characterised in that** the soluble bases with organic solvents can be used as the base and that their amounts ensure the bonding of the acid being deposited.

11. Method according to claim 1, **characterised in that** the base used is N-methylmorpholine.

12. Method according to claim 1, **characterised in that** triethylamine is used as the base.

13. Method according to claim 1, **characterised in that** diisopropylethylamine is used as the base.

14. Method according to claim 1, **characterised in that** dimethylacetamide, N-methylpyrrolidone, dimethyl sulfoxide or dimethylformamide are used as the organic solvent.

## Revendications

1. Procédé de fabrication de créatinamides sous forme de sels, **caractérisé par** les étapes de procédé suivantes :
- le traitement de créatine avec de l'acide p-toluène-sulfonique dans un solvant organique, puis
- l'interaction du complexe formé avec des dérivés d'acides aminés contenant un groupe amino primaire ou secondaire, en présence d'un agent actif de condensation introduit ultérieurement, de préférence un carbodiimide ou un ester de l'acide chloroformique, et d'une base, de préférence une amine tertiaire.

2. Procédé selon la revendication 1, **caractérisé en ce que** le traitement avec l'acide p-toluène-sulfonique a lieu au moins en une quantité équimolaire par rapport à la créatine.

3. Procédé selon la revendication 1, **caractérisé en ce que** de la créatine anhydre est utilisée en tant que créatine.

4. Procédé selon la revendication 1, **caractérisé en ce que** du monohydrate de créatine est utilisé en tant que créatine.

5. Procédé selon la revendication 1, **caractérisé en ce que** des dérivés ester ou amide d'acides aminés aliphatiques ou aromatiques sont utilisés en tant que dérivés d'acides aminés.

6. Procédé selon la revendication 1, **caractérisé en ce que** le dicyclohexylcarbodiimide est utilisé en tant qu'agent actif de condensation.

7. Procédé selon la revendication 1, **caractérisé en ce que** le diisopropyl-carbodiimide est utilisé en tant qu'agent actif de condensation.

8. Procédé selon la revendication 1, **caractérisé en ce que** l'ester isobutylique de l'acide chloroformique est utilisé en tant qu'agent actif de condensation.

9. Procédé selon la revendication 1, **caractérisé en ce que** de l'ester éthylique de l'acide chloroformique est utilisé en tant qu'agent actif de condensation.

10. Procédé selon la revendication 1, **caractérisé en ce que** des bases solubles dans des solvants organiques sont utilisées en tant que bases et **en ce que** leurs quantités assurent la liaison de l'acide qui se sépare.

11. Procédé selon la revendication 1, **caractérisé en ce que** la N-méthylmorpholine est utilisée en tant que base.

12. Procédé selon la revendication 1, **caractérisé en ce que** la triéthylamine est utilisée en tant que base.

13. Procédé selon la revendication 1, **caractérisé en ce que** la diisopropyléthylamine est utilisée en tant que base.

14. Procédé selon la revendication 1, **caractérisé en ce que** le diméthylacétamide, la N-méthylpyrrolidone, le diméthylsulfoxyde ou le diméthylformamide est utilisé en tant que solvant organique.
